Europäisches Patentamt

European Patent Office

Office européen des brevets

(19)

(11) Publication number: **0 142 301 B1**

(12) ## EUROPEAN PATENT SPECIFICATION

(45) Date of publication of patent specification: 27.11.91   (51) Int. Cl.⁵: **G01N 33/53**, G01N 33/543, G01N 33/542

(21) Application number: 84307300.8

(22) Date of filing: 24.10.84

(54) **Methods of assay.**

(30) Priority: 25.10.83 GB 8328520

(43) Date of publication of application:
22.05.85 Bulletin 85/21

(45) Publication of the grant of the patent:
27.11.91 Bulletin 91/48

(84) Designated Contracting States:
AT BE CH DE FR GB IT LI NL SE

(56) References cited:
EP-A- 0 078 636
WO-A-80/02201
GB-A- 1 543 097
US-A- 4 334 880

CLINICAL CHEMISTRY, vol. 28, no. 9, 1982,
Washington (US); K.R.WEHMEYER et al.:
"Electrochemical investigation of hapten-
antibody interactions by differential pulse
polarography", pp. 1968-1972

CHEMICAL ABSTRACTS, vol. 91, no. 1, 02
July 1982, Columbus, OH (US); S.G.WEBER et
al., p. 221, no. 2126y

CHEMICAL ABSTRACTS, vol. 97, no. 12, 20
Sep 1982, Columbus, OH (US); p. 794, no.
103684h

(73) Proprietor: SERONO DIAGNOSTICS LIMITED
21 Woking Business Park Albert Drive
Woking Surrey GU21 5JY(GB)

(72) Inventor: **Forrest, Gordon Coulter**
**Braemore High Park Avenue**
**East Horsley Surrey KT24 5DP(GB)**
Inventor: **Robinson, Grenville Arthur**
**23, Burnham Way**
**Ealing London, W13 9YE(GB)**
Inventor: **Hill, Hugh Allen Oliver**
**9, Clover Close**
**Oxford(GB)**

(74) Representative: **Woodman, Derek et al**
**Frank B. Dehn & Co. European Patent Attor-**
**neys Imperial House 15-19 Kingsway**
**London WC2B 6UZ(GB)**

EP 0 142 301 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 142 301 B1

## Description

The present invention relates to methods of assay of one of a pair of specific binding partners, and to kits of reagents for carrying out these methods.

There is today a great need for rapid and accurate methods of assaying biologically active substances (which may be at low concentration), particularly in body fluids such as blood, saliva or urine. A wide variety of medical conditions, such as pregnancy, drug overdose, metabolic birth defects, hormonal disorders and diabetes can be diagnosed using such assay techniques.

Many assay methods rely on the formation of a complex between the species under assay (hereinafter called "ligand") and another species to which it will bind specifically (hereinafter called "specific binding partner"). The extent of complex formation is a function of the amount of the ligand present.

The assay of ligand is determined by monitoring the extent of complex formation, for example by the use of chemical or biochemical labels. Several methods of labelling have been employed, for example radioisotopic or enzyme labelling, spin-labelling or labelling employing fluorescent or bioluminescent species. Although each of these known methods may be advantageous in certain respects, it has been found in practise that, in general, only radioisotopic labelling gives a method which is sufficiently rapid, sensitive and specific, and thus it is that technique which has become most widely used.

There are, however, serious disadvantages to the use of radioactive labels. Radioactive labels have limited shelf life due to spontaneous decay, necessitating frequent recalibration of the equipment, and their use will require adherence to strict safety precautions and is subject to legal regulation.

Furthermore, a separation step may be necessary following the formation of the labelled complex in order to remove unbound labelled component from the system before determination of the amount of labelled complex by the usual counting methods.

We have now found that, by employing a redox centre as herein defined as a label and measuring a perturbation in an electrochemical characteristic of the components, a simple, rapid, sensitive and specific method of quantitative or qualitative ligand assay may be obtained, which avoids the use of radioactive species.

Thus, in its broadest aspect, the invention provides a method of assay of a ligand in a sample using an electrochemical apparatus containing components comprising

(a) the sample,

(b) a specific binding partner to the ligand, and

(c) if desired, at least one reagent selected from ligand analogues and specific binding partners to the ligand,

at least one of the said components other than the sample being labelled with at least one redox centre selected from ferrocene and derivatives thereof;

which method includes the step of determining whether, and, if desired, the extent to which an electrochemical characteristic of the components in a quiescent solution is perturbed by at least one of (i) complex formation and (ii) a controlled external influence which produces a perturbation of said electrochemical characteristic as a function of said complex formation.

The assay can be completed from the determined perturbation with reference to calibration data.

The term "redox-modified" used herein refers to the modification of a molecular structure by incorporation of at least one redox centre. The term redox centre as used herein refers to a species selected from ferrocene and derivatives thereof capable of undergoing, under suitable conditions, reduction and oxidation. Typically, the redox-centre will undergo both oxidation and reduction during the period of the assay, thereby returning at the end of the assay to its initial oxidation level.

The term "ligand analogue" used herein refers to a species capable of complexing with the same specific binding partner as the ligand under assay, and includes inter alia within its scope a known quantity of the ligand species under assay.

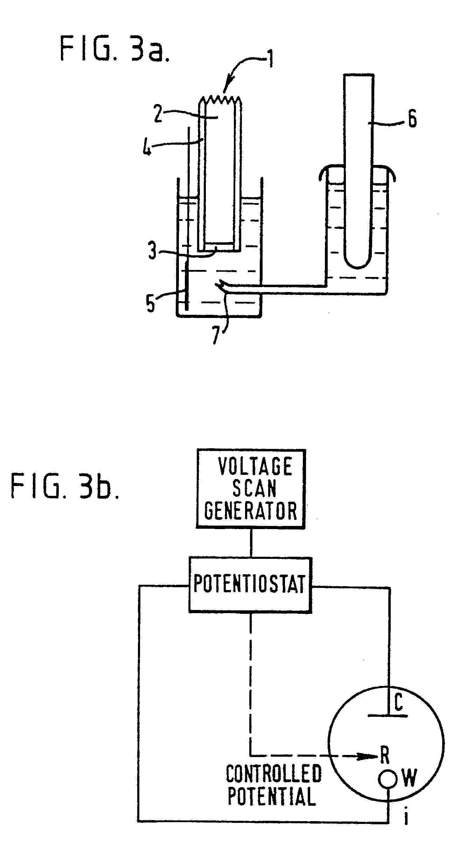

The electrochemical apparatus contains a working electrode, an auxiliary electrode and optionally a reference electrode. The working electrode will preferably be solid and have a working surface of e.g. carbon (preferably graphite e.g. pyrolytic graphite), or metal e.g. silver, gold or platinum. A suitable electrochemical apparatus is illustrated in Figure 3a of the accompanying drawings. The working electrode 1 is composed of an elongated core 2 (e.g. of brass) tipped with a working surface 3 (e.g. of pyrolytic graphite) and having a coating 4 (e.g. of epoxy resin). The auxiliary (counter) electrode 5 is suitably of platinum. A calomel reference electrode 6 is shown, connected to the cell via a luggin capillary 7.

Although the order of introduction of the components into the apparatus is not critical, it is preferable that a complex is formed after introduction of the final component but not prior thereto. It is, however, also possible for there to be complex present before the final component is added, in which case the final

2

component will become complexed by displacing one component of the complex.

A variety of electrochemical methods exploiting any two of the three parameters potential (E), current (i) and time (t) may be used to measure electrochemical characteristics of the components. For example, electrochemical measurements can be made using differential pulse voltammetry, cyclic voltammetry or square-wave voltammetry. When cyclic voltammetry is used, a circuit such as, for example, that shown in Figure 3b of the accompanying drawings may be employed. In this Figure, C represents the auxiliary (counter) electrode, W the working electrode and R the reference electrode. This circuit may conveniently be used in conjunction with apparatus of the type shown in Figure 3a, the electrochemical current i being measured.

In a typical case, formation of the complex perturbs the electrochemical behaviour of the redox centre, the degree of perturbation being a function of the proximity of the site of complex formation to the redox centre. It is not necessary for a full voltammogram to be determined in the measurements made; it may be sufficient, for example, for an appropriate poised potential to be selected and readings of current taken at that point. The degree or rate of perturbation can then be related to the amount of ligand present in the sample, from calibration data obtained with similar systems using known amounts of ligand.

It may be found, on measuring the perturbation of an electrochemical characteristic, that no, or only a slight, perturbation is observed due to complex formation. In that case, it may prove advantageous artificially to generate or enhance a perturbation by controlled external influences. Although the magnitude of the external influence may have some bearing on the change induced, and must therefore be consistent with any such influence employed in calibration experiments, it is thought that any change produced in the perturbation remains a function of the ligand/binding partner complex.

The artificial generation or enhancement of the perturbation is preferably performed by displacement of the complex relative to the working electrode, for example by further complexing the complex with a species which will bind specifically to the complex, coupled to a solid support, or by using a specific binding partner coupled to a solid support, with subsequent displacement of the support and coupled molecules. In extreme cases, the displacement may constitute complete removal of the complex from the apparatus, but in general the complex will be displaced within the apparatus. Other methods for artificially generating or enhancing the perturbation include, for example, the use of antibodies to the redox-centre to quench the electrochemical characteristic of the redox-centre, or removing excess uncomplexed redox-modified reagent, e.g. by draining from the apparatus, or by coupling to a suitable solid support and removing the said solid support from the apparatus.

In a method of the invention exploiting the time (t) parameter, the rate of perturbation of the electrochemical characteristic as a result of complex formation may be determined. Conveniently, the initial rate of perturbation will be measured. Such a method is applicable to a competitive assay in which the ligand and a redox-modified ligand analogue compete for complexing with the specific binding partner. The reaction may be viewed as competitive inhibition of the ligand complexing reaction by the ligand analogue. Thus, the initial rate of perturbation is related to the concentration of ligand present and from a calibration plot of the initial rate of perturbation v. concentration of ligand present, the ligand assay can be readily determined.

The method of assay involving a determination of the rate of perturbation is also applicable to non-competitive assays where the redox-modified ligand analogue is absent and sufficient redox-modified specific binding partner is employed to enable all the ligand introduced to be complexed. Artifical generation or enhancement of the perburbation as herein described may if desired be carried out before the rate of perturbation is determined.

In another competitive assay, not involving a rate determination, an initial reading of the appropriate electrochemical characteristic will be made before the addition of a final component which may initiate the complexing reaction or may displace a component from a complex already present. On completion of complex formation, a final reading of the electrochemical characteristic of the components will be taken and the perturbation determined. By reference to calibration data of perturbations with known ligand concentrations the amount of ligand in the sample can be determined.

The method of the invention also enables a non-competitive assay, not involving a rate determination, to be performed.

So long as there is sufficient specific binding partner present, the absolute perturbation, or the rate of perturbation, in the electrochemical characteristic of the components from the initial reading before introduction of the final component will be dependent on the amount of added ligand and the assay can therefore be calculated with reference to calibration data.

The methods of the present invention are generally simpler than known methods, in that they may eliminate the need for the separation of complexed and uncomplexed phases before the assaying step.

If electrode-immobilised components are employed, the technique is further simplified in that the need for separate additon of the component to the electrochemical apparatus is eliminated. Additionally, the direct interaction between the electrode and the electrode-immobilised species may lead to an improvement in the sensitivity of the perturbation measurements.

According to one embodiment of the present invention, therefore, there are provided methods of assay of a ligand in a sample as hereinbefore defined wherein the working electrode of the electrochemical apparatus is solid and one of the components other than the sample is immobilised thereon.

The immobilised component may thus be a redox-modified or unmodified ligand analogue or specific binding partner.

It will be appreciated that, in this embodiment, due to the resulting complex being coupled to the electrode, it is not possible artificially to generate or enhance a perturbation by displacement of the resulting complex relative to the working electrode. However, a perturbation may still be artificially generated or enhanced, for example by complexing any uncomplexed redox-modified reagent remaining in solution with a species which will complex specifically with the reagent, coupled to a solid support, with subsequent displacement of the support and coupled molecules.

The invention will be particularly described hereinafter with reference to an antibody or an antigen as the ligand. However the invention is not to be taken as being limited to assays of antibodies or antigens. Examples of ligands which may be assayed by the method of the invention are given in Table I below, together with an indication of a suitable specific binding partner in each instance.

## Table I

| Ligand | Specific Binding Partner |
|---|---|
| antigen | specific antibody |
| antibody | antigen |
| hormone | hormone receptor |
| hormone receptor | hormone |
| polynucleotide strand | complementary polynucleotide strand |
| avidin | biotin |
| biotin | avidin |
| protein A | immunoglobulin |
| immunoglobulin | protein A |
| enzyme | enzyme cofactor (substrate) |
| enzyme cofactor (substrate) | enzyme |
| lectins | specific carbohydrate |
| specific carbohydrate of lectins | lectins |

The method of the invention has very broad applicability but in particular may be used to assay: hormones, including peptide hormones (e.g. thyroid stimulating hormone (TSH), luteinising hormone (LH), follicle stimulating hormone (FSH), human chorionic gonadotrophin (hCG), insulin and prolactin) or non-peptide hormones (e.g. steroid hormones such as cortisol, estradiol, progesterone and testosterone, or thyroid hormones such as thyroxine (T4) and triiodothyronine), proteins (e.g. carcinoembryonic antigen (CEA) and alphafetoprotein (AFP)), drugs (e.g. digoxin), sugars, toxins or vitamins.

It will be understood that the term "antibody" used herein includes within its scope

a) any of the various classes or sub-classes of immunoglobulin, e.g. IgG, IgM, derived from any of the animals conventionally used, e.g. sheep, rabbits, goats or mice,

b) monoclonal antibodies,

c) intact molecules or "fragments" of antibodies, monoclonal or polyclonal, the fragments being those which contain the binding region of the antibody, i.e. fragments devoid of the Fc portion (e.g., Fab, Fab', $F(ab')_2$) or the so-called "half-molecule" fragments obtained by reductive cleavage of the disulphide bonds connecting the heavy chain components in the intact antibody.

The method of preparation of fragments of antibodies is well known in the art and will not be described herein.

The term "antigen" as used herein will be understood to include both permanently antigenic species (for example, proteins, bacteria, bacteria fragments, cells, cell fragments and viruses) and haptens which may be rendered antigenic under suitable conditions.

Incorporation of the redox centre into the molecular structure of an antibody may be achieved, for example, by any of the following methods:

(i) providing the redox centre with one or more functional groups capable of bonding interactions with the molecular structure of the antibody;

(ii) using cross-linking groups;

(iii) using the avidin-biotin binding system, (e.g. avidin-labelled antibody binding with biotin-labelled ferrocene molecules or biotin-labelled antibody binding with avidin-labelled ferrocene);

(iv) using an antibody conjugated with a reagent (e.g. fluorescein isothiocyanate (FITC)) binding with a second antibody raised to the reagent (e.g. anti-FITC antibody) to which is coupled ferrocene;

(v) using a second antibody labelled with ferrocene,

said second antibody being raised to the first antibody.

Similar methods may be applied as desired for the incorporation of a redox centre into an antigen molecule. Suitable methods are known in the art and will not be discussed in detail here. For example, the incorporation of ferrocene into certain steroids was described in Journal of Organometallic Chemistry, 160 - (1978) pp. 223-230.

Immobilisation of an antibody or antigen molecule onto the electrode may be effected by various methods. The attachment of the antibody or antigen to the electrode can be via any portion of the molecular structure so long as specific immunological activity is retained at the antibody or antigen binding site.

Thus, for example, electrode-immobilisation of unmodified antibody or antigen reagent may be achieved by bonding interactions between functional groups on the antibody or antigen molecule and the electrode, or by cross-linking or adsorption onto the surface of the electrode. Binding of reagents to the electrode may be accomplished by methods analogous to known methods for binding such reagents to solid supports (e.g. particles and beads), for example those described in published European Patent Application No. 105714.

Electrode-immobilisation of redox-modified reagent may, for example, be achieved by any of the following methods:

(i) incorporating a redox centre into the molecular structure of free reagent and subsequently immobilising the reagent onto an electrode at a site remote from the redox centre in the same way as described above for unmodified reagents;

(ii) incorporating a redox centre into the molecular structure of a pre-immobilised reagent;

(iii) incorporating a bifunctional redox centre into the molecular structure of free antibody or antigen so as to enable one function to interact with the electrode: or

(iv) incorporating a bifunctional redox centre onto the electrode, so as to enable one function to interact with the molecular structure of free antibody or antigen.

The organometallic 'sandwich' compound ferrocene (bis-$\eta^5$-cyclopentadienyl iron (II)) or a derivative thereof is desirable as a redox centre label because it is relatively cheap, non-toxic and provides an easily reversible system which in its reduced $Fe^{II}$ state is not susceptible to oxidation by oxygen in the atmosphere. Examples of suitable ferrocene derivatives include functionalised derivatives, polymeric forms ('polyferrocenes') such as $(ferrocene)_4$ and 'boron tetraferrocene' ($B(ferrocene)_4$).

As indicated above, the redox centre may require functionalisation in order to permit successful binding to the antibody or antigen and/or to the electrode or to improve the electrochemical or other propeties of the redox centre. For example, the redox potential of ferrocene is +442 mV vs NHE. By introducing functional groups onto the ring system, this figure can be varied between +300 and +650 mV. Moreover, the water-solubility of carboxyl-substituted ferrocene is greater than that of the parent compound (see, for example, R. Szentrinay 1977 Amer. Chem. Soc. Symposium Series, 38, 154).

Thus, for example, in the case of ferrocene, it may be necessary to modify the ferrocene complex by providing one or both of the cyclopentadienyl groups with one or more side chains, e.g. of the formula

-CHO
$-(CH_2)_nCOOH$ or
$-(CH_2)_mNH_2$

where n and m may be e.g. from 0 to 6. Additional functional groups may be incorporated into the side chain, typically those groups used in the chemical modification of proteins, for example mercuric chloride, precursors of nitrenes and carbenes, diazo or iodide groups. The terminal -CHO, -COOH or $-NH_2$ groups are then available to interact with suitable sites in the antibody or antigen molecule or on the electrode. The length of the side chain (i.e. the value of n or m) will depend on the structure of the antibody or antigen to which the ferrocene is to be bound.

The electrochemical apparatus preferably contains an aqueous assay medium comprising inter alia, pH buffer. Means may be provided for incubating the assay mixture at any desired temperature.

In a further feature of the present invention, therefore, there are provided kits of reagents and apparatus for carrying out the assays of the invention. Suitable kits may comprise an electrochemical apparatus containing at least one electrode; and an aqueous assay medium with suitable redox-modified or unmodified reagent present (either in solution or immobilised on the working electrode). Other components (e.g. further reagents etc) and the sample to be assayed may conveniently be introduced through an entry port provided in the apparatus.

Advantageously the apparatus may be pre-calibrated and provided with a scale whereby the perturbation in electrochemical characteristic of the components may be read off directly as an amount of antibody or antigen in the sample.

In kits employing the competitive assay method, the competing redox-modified ligand analogue reagent may preferably be initially mixed with the sample to be assayed outside the apparatus and the mixture then introduced into the apparatus.

As illustration of various aspects of this invention, antibody and antigen assays which may be performed according to the invention are given in Table II below. These assays are given purely for illustration and are in no way limiting of the present invention. It will be appreciated that the assays give in Table II may, where appropriate, be carried out either by an absolute or by a rate method as described above.

Ab = antibody
Ag = antigen
* = redox-modified species
— = species under assay (in sample
(lim) = known, limited amount of reagent

## Table II

### A. Assay of Antigen

| Components of apparatus immediately after initial measurement of electro-chemical characteristic | Subsequent assay steps |
|---|---|
| 1.<br><br>[ELECTRODE]   Ab* | a) <u>Ag</u> added to form Ab*:<u>Ag</u> complex<br>b) optional generation or enhancement of perturbation<br>c) measurement of electro-chemical characteristic<br>d)  calculation of assay from perturbation |
| 2.<br><br>[ELECTRODE]   <u>Ag</u> | a) Ab* added to form Ab*:<u>Ag</u> complex<br>b) optional generation or enhancement of perturbation<br>c) measurement of electro-chemical characteristic<br>d)  calculation of assay from perturbation |
| 3.<br><br>[ELECTRODE]—Ab* | a) <u>Ag</u> added to form immobilised Ab*:<u>Ag</u> complex<br>b) measurement of electro-chemical characteristic<br>c)  calculation of assay from perturbation |

7

| | |
|---|---|
| 4.<br><br>[ELECTRODE]—Ab   Ab* | a) Ag (having two roomly-spaced determinants to which the antibody reagents are separately directed) added to form [ELECTRODE]—Ab:Ag:Ab* complex<br>b) optional generation or enhancement of perturbation<br>c) measurement of electrochemical characteristic<br>d) calculation of "two-site" assay from perturbation |
| Note: This assay method may be carried out using the "forward", "simultaneous" or reverse" techniques. | |
| 5.<br><br>[ELECTRODE]—Ag*(lim)<br>           Ag | a) Ab (lim) added to give a competitive system<br>b) measurement of electrochemical characteristic<br>c) calculation of assay from perturbation. |
| 6. [ELECTRODE]—Ab(lim) | a) Ag*(lim) and Ag added together to give competitive system<br>b) optional generation or enhancement of perturbation<br>c) measurement of electrochemical characteristic<br>d) calculation of assay from perturbation |

| 7. [ELECTRODE] Ab(lim) | a) <u>Ag</u> and Ag* added to give a competitive system<br>b) optional generation or enhancement of perturbation<br>c) measurement of electro-chemical characteristic<br>d) calculation of assay from perturbation |
|---|---|
| 8. [ELECTRODE]—Ag <u>Ag</u> | a) Ab* (lim) added to give a competitive system<br>b) generation or enhancement of perturbation<br>c) measurement of electro-chemical characteristic<br>d) calculation of assay from perturbation |

### B. Assay of Antibody

| Components of apparatus immediately after initial measurement of characteristic | Subsequent assay steps |
|---|---|
| 9.<br><br>  [ELECTRODE]      Ag* | a) <u>Ab</u> added to form <u>Ab</u>:Ag* complex<br>b) optional generation or enhancement of perturbation<br>c) measurement of electro-chemical characteristic<br>d) calculation of assay from perturbation |
| 10.<br><br>  [ELECTRODE]      <u>Ab</u> | a) Ag* added to form <u>Ab</u>:Ag* complex<br>b) optional generation or enhancement of perturbation<br>c) measurement of electro-chemical charcteristic<br>d) calculation of assay from perturbation |
| 11.<br><br>  [ELECTRODE]—Ag* | a) <u>Ab</u> added to form immobilised <u>Ab</u>:Ag* complex<br>b) measurement of electro-chemical characteristic<br>c) calculation of assay from perturbation |

| 12.<br><br>[ELECTRODE]—Ab   <u>Ab</u><br><br><br><br><br><br><br><br><br><br>Note: This assay method may be carried out using the "forward", "simultaneous" or "reverse" techniques. | a) Ag* (having two roomly-spaced determinants to which the antibody reagents are separately directed) added to form [ELECTRODE]—Ab:Ag*:<u>Ab</u> complex.<br>b) measurement of electro-chemical characteristic<br>c) calculation of "two-site" assay from perturbation |
| 13.<br><br>[ELECTRODE]   Ab*(lim)<br>              <u>Ab</u> | a) Ag(lim) added to give competitve system<br>b) measurement of electro-chemical characteristic<br>c) calculation of assay from perturbation. |
| 14.<br>   [ELECTRODE]—Ab*(lim)<br>                  <u>Ab</u> | a) Ag(lim) added to give competitive system<br>b) measurement of electro-chemical characteristic<br>c) calculation of assay from perturbation |

| 15.<br><br>   [ELECTRODE]—Ag(lim) | a) Ab*(lim) and Ab added together to give competitive system<br>b) optional generation or enhancement of perturbation<br>c) measurement of electro-chemical characteristic<br>d) calculation of assay from perturbation |
|---|---|
| 16.<br><br><br>   [ELECTRODE]     Ag(lim) | a) Ab and Ab* (lim) added to give a competitive system<br>b) optional generation or enhancement of perturbation<br>c) measurement of electro-chemical characteristic<br>d) calculation of assay from perturbation |
| 17.<br><br>   [ELECTRODE]—Ab     Ab | a) Ag* (lim) added to give a competitive system<br>b) optional generation or enhancement of perturba-tion<br>c) measurement of electro-chemical characteristic<br>d) calculation of assay from preturbation |

The following non-limiting Examples are intended to illustrate the invention more fully

EXAMPLE 1

Assay of Thyroxine (T4) using a redox-modified antigen with enhancement of perturbation using controlled external influences

In this assay free redox-modified antigen is measured after competition between modified and unmodified antigen for a fixed number of antibody binding sites.

Preparation of Starting Materials

## (i) Ferrocene-modified thyroxine (T4) hormone

To stirred, cooled (-10°C) dimethylformamide (3 mls), was added ferrocene monocarboxylic acid (FMCA) (1 m mole) and then triethylamine (1.0 m mole) and isobutylchloroformate (1.0 m mole). The mixture was then further cooled to -15°C and stirred for 30 minutes and then allowed to warm up to room temperature and stirred for a further hour. The resulting carboxycarbonic anhydride of ferrocene was added dropwise to a solution of T4 in sodium hydroxide/ethanol and stirred for 24 hours, after which the sample was filtered and then concentrated by rotary evaporation. The resulting precipitate was washed in carbon tetrachloride, then ethyl acetate several times, the insoluble ferrocene monocarboxylic acid-T4 conjugate (T4-FMCA) then being filtered out of the ethyl acetate solution.

## (ii) Purification of Ferrocene-modified thyroxine conjugate (T4-FMCA)

The T4-FMCA conjugate was purified by high performance liquid chromatography using C18 reverse phase column. After purification the T4 and ferrocene concentrations in the conjugate were measured by radioimmunoassay and electrochemistry respectively.

## (iii) Characteristics of the T4-FMCA conjugate

Incubation of T4-FMCA with excess anti-T4 antibody showed that 73. 4% of the T4-FMCA was bound to the antibody.

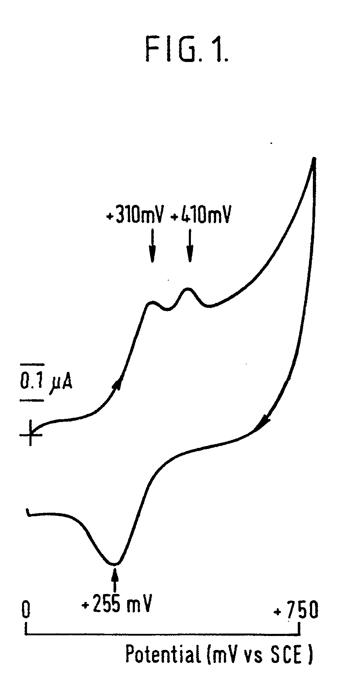

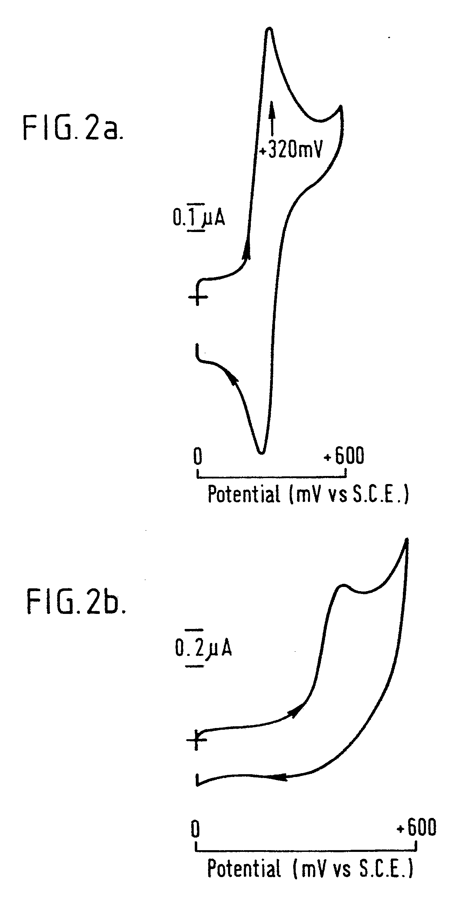

The electrochemistry of the conjugate was also compared with that of the T4 and FMCA. The conjugate shows two peaks on the forward wave of the cyclic voltammogram at a pyrolytic graphite electrode (Voltage Scan Rate = 20mVs$^{-1}$), the first being at +310 mV vs a standard calomel electrode (S.C.E.) (the ferrocene peak), the second at +410 mV vs S.C.E. (T4) (Figure 1). On the return wave only one peak at a potential of +255 mV vs S.C.E. was observed. Cyclic voltammograms at pyrolytic graphite electrodes (Voltage Scan Rates = 5mVs$^{-1}$) of the unmodified components of the conjugate show that FMCA (0.2 mM FMCA in Tris (50mM; pH 7.4)) has peaks at +320 mV and +260 mV vs S.C.E. for the forward and return waves respectively (Figure 2a) whilst T4 exhibits a single peak at +420 mV vs S.C.E. on the forward wave (Figure 2b).

## (iv) Solid Phase reagent comprising anti-T4 polyclonal antibodies covalently linked to magnetisable cellulose particles.

Anti-T4 was a conventional polyclonal antiserum obtained by immunising sheep with T4 conjugated to a high molecular weight protein. The magnetisable cellulose particles were a composite of cellulose containing approximately 50% black ferric(ous) oxide ($Fe_3O_4$), with mean particle diameter of 3 micron (see Forrest and Rattle, "Magnetic Particle Radioimmunoassay", in Immunoassays for Clinical Chemistry, p. 147-162, Ed. Hunter and Corrie, Churchill Livingstone, Edinburgh (1983)). Anti-T4 antiserum was covalently coupled to the magnetisable cellulose following cyanogen bromide activation of the cellulose, according to the procedure of Axen et al, Nature 214, 1302-1304 (1967). The antiserum was coupled at a ratio of 1 ml antiserum to 1 gram of magnetisable solid phase. Anti-T4 magnetisable solid phase was diluted to a concentration of 25 g/ml in Tris-HCl buffer, 50 mM, pH 7.4.

## (v) Apparatus used to measure the electrochemistry of T4-FMCA.

Cyclic voltammetry was performed in a three electrode electrochemical cell using a pyrolytic graphite working electrode (Figures 3a and 3b).

## (vi) Standard solutions of T4

T4 (sodium salt) was obtained from Sigma London Chemical Company, England. Standard solutions were made by dissolving the T4 in sodium hydroxide (0.1 M) and then diluting with Tris-HCl buffer (50 mM pH 7.4) to the desired concentration.

## (vii) Assay procedure for T4

Duplicate samples were run, in which 50 μl of T4 standard was added to 100 μl of T4-FMCA (1.3 x

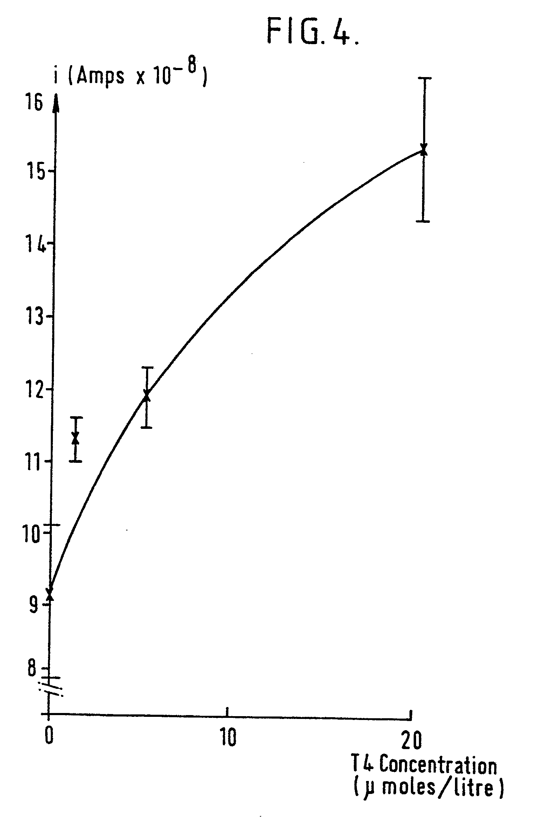

$10^{-5}$ M) and mixed, after which 100 $\mu$l anti-T4 antibody coupled to solid phase was added. The mixture was incubated at room temperature for 30 minutes with constant agitation and then the solid phase particles were separated magnetically. The resulting supernatant was transferred to the electrochemical cell where the current at a potential of +310 mV vs standard calomel electrode (S.C.E.) was measured. An example current/T4 concentration curve is shown in Figure 4. Electrochemical Current i in amps x $10^{-8}$ is plotted along the vertical axis whilst T4 concentration (in $\mu$ moles per litre) is plotted along the horizontal axis.

EXAMPLE 2

Assay of human chorionic gonadotrophin (hCG) using a redox-modified antibody with enhancement of perturbation using controlled external influences

Preparation of starting materials

(i) Redox-modified anti-hCG monoclonal antibodies

Monoclonal antibodies were obtained from mouse ascites fluid by the process reported by Milstein and Kohler in Nature 256 495-497 (1975). Antibodies from individual hybridoma cell lines were screened to identify those producing antibody to discrete antigenic determinants. Those having the highest affinities to hCG were selected for use in the assay.

Ferrocene carboxaldehyde (FCA) was used to introduce a redox centre into the structure of an anti-hCG monoclonal antibody, antibody A. The incorporation of the FCA into the antibody was achieved by formation of a Schiff's base between the active aldehyde group of the ferrocene and a free amino group on the antibody.

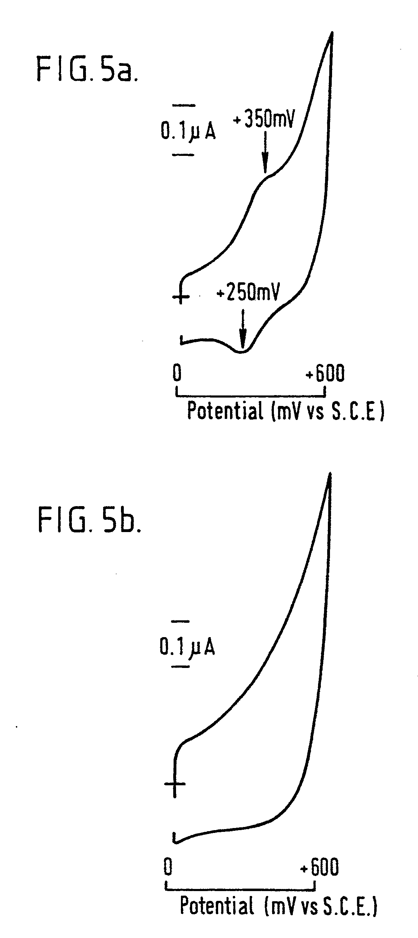

Ferrocene carboxaldehyde (0.15 mg per ml in sodium carbonate buffer 1 M per litre, pH 9.5) was incubated with 0.27 mg per ml of antibody A (also in sodium carbonate buffer) for 2 hours at 22°C in the dark with constant mixing. The resulting conjugate was purified by gel filtration. The fractions were examined electrochemically and only those fractions whose cyclic voltammogram at a pyrolytic graphite electrode (with a Voltage Scan of 10mVs$^{-1}$) exhibited the forward and return waves characteristic of the ferrocene redox reaction (as shown in Figure 5a of the accompanying drawings) were used in the assay, those antibody fractions having no incorporated ferrocene (see Figure 5b) being discarded.

(ii) Preparation of anti-hCG (antibody B) conjugated to fluorescein isothiocyanate (FITC)

A second monoclonal antibody to hCG (antibody B) specific for a different antigenic determinant was conjugated to FITC.

Conjugation of FITC to monoclonal antibody was achieved by reacting 200 $\mu$g fluorescein isothiocyanate (FITC), Sigma London Chemical Co., England, with 5 mg antibody in 1.4 ml sodium bicarbonate buffer, 0.2 M, pH 9.0, for 18 hours at room temperature. The reaction mixture was purified by gel filtration on Sephadex G-50 superfine, giving a product incorporating an average of 6 molecules FITC per antibody molecule.

(iii) Preparation of anti-FITC antibody covalently coupled to magnetisable solid phase

Anti-FITC was a conventional polyclonal antiserum obtained by immunising sheep with FITC conjugated to keyhole limpet haemocyanin. The magnetisable cellulose particles were a composite of cellulose containing approximately 50% black ferric(ous) oxide ($Fe_3O_4$), with mean particle diameter of 3 micron (see Forrest and Rattle, "Magnetic Particle Radioimmunoassay", in Immunoassays for Clinical Chemistry, p. 147-162, Ed. Hunter and Corrie, Churchill Livingstone, Edinburgh (1983)). Anti-FITC antiserum was covalently coupled to the magnetisable cellulose following cyanogen bromide activation of the cellulose, according to the procedure of Axen et al, Nature 214, 1302-1304 (1967). The antiserum was coupled at a ratio of 2 ml antiserum to 1 gram of magnetisable solid phase.

Anti-FITC magnetisable solid phase was diluted to 100 mg per ml in Tris-HCl buffer (10 mM per litre, pH 7.4).

(iv) Preparation of hCG standard solutions

A freeze dried preparation of hCG, calibrated against the first international reference preparation (75/537) was obtained from Biodata S.p.A., Milan, Italy. This sample was diluted in buffer Tris-HCl, (10 mM, pH 7.4) to the desired concentration.

(v) Apparatus used for electrochemical measurement

The electrochemical apparatus was the same equipment as that of Example 1.

(vi) Assay procedure for hCG

An immunometric immunoassay using the redox-modified antibody A was used to measure hCG.

Fixed volumes (100 $\mu$l) of hCG standard were mixed with 100 $\mu$l of antibody A (10 $\mu$g per ml protein) and 100 $\mu$l of antibody B (6 ug per ml protein). After a 30 minute incubation period at room temperature the electrochemical current was measured (t=0) after which 20 $\mu$l of anti-FITC antibody was added and incubated for a further five minutes with constant agitation. The application of an external magnetic field removed the solid phase and attached immunocomplex from solution, the electrochemical current due to any ferrocene remaining in the supernatant being measured (t=5).

The electrochemical signal was defined as:

$$\text{signal unit} = \frac{(i_{t=0}) - (i_{t=5}) \text{ for sample}}{(i_{t=0}) - (i_{t=5}) \text{ for zero standard}}$$

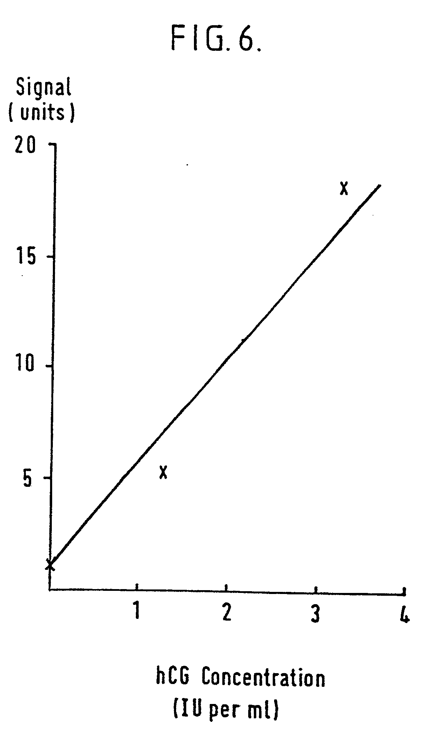

where i = electrochemical current measured at +330 mV vs S.C.E.. The zero standard was an analagous system containing no hCG. A plot of signal versus hCG concentration (in International Units per ml) is shown in Figure 6 of the accompanying drawings.

## Claims

1. A method of assay of a ligand in a sample using an electrochemical apparatus containing components comprising
   (a) the sample
   (b) a specific binding partner to the ligand, and
   (c) if desired, at least one reagent selected from ligand analogues and specific binding partners to the ligand,
   at least one of the said components other than the sample being labelled with at least one redox centre selected from ferrocene and derivatives thereof;
   which method includes the step of determining whether, and, if desired, the extent to which an electrochemical characteristic of the components in a quiescent solution is perturbed by at least one of (i) complex formation and (ii) a controlled external influence which produces a perturbation of said electrochemical characteristic as a function of said complex formation..

2. A method as claimed in claim 1, wherein the electrochemical apparatus contains a solid working electrode.

3. A method as claimed in claim 1 or claim 2, wherein the controlled external influence, when present, comprises displacement of the complex formed relative to the working electrode of the electrochemical apparatus, or removal of the complex from the apparatus.

4. A method as claimed in claim 2, wherein one of the components other than the sample is immobilised on the working electrode.

5. A method as claimed in any one of claims 1, 2 and 4, wherein the controlled external influence, when present, comprises displacement of excess uncomplexed redox-modified component relative to the working electrode of the electrochemical apparatus, or removal of excess uncomplexed redox-modified component from the apparatus.

6. A method as claimed in any preceding claim, wherein the or each redox-modified component contains at least one redox centre selected from functionalised derivatives of ferrocene.

7. A method as claimed in claim 6, wherein the functionalised derivative of ferrocene contains one or more side chains of the formula -CHO, $-(CH_2)_nCOOH$ or $-(CH_2)_mNH_2$ (where n and m are each from 0 to 6).

8. A method as claimed in any preceding claim, wherein the electrochemical characteristic is a peak current observed under the application of a preselected potential across the components.

9. A method as claimed in any preceding claim wherein the ligand is an antigen or an antibody.

10. A kit for carrying out a method of assay as claimed in claim 1 which comprises a reagent selected from specific binding partners to the ligand and ligand analogues labelled with ferrocene or a derivative thereof, said reagent being immobilised on a solid working electrode.

11. A kit for carrying out a method of assay as claimed in claim 1 which comprises an unlabelled specific binding partner to the ligand immobilised on a solid support and a further reagent selected from ligand analogues and specific binding partners to the ligand labelled with ferrocene or a derivative thereof.

**Revendications**

1. Procédé d'essai d'un ligand dans un échantillon utilisant un appareil électrochimique contenant des composants comprenant:
   (a) l'échantillon,
   (b) un partenaire de liaison spécifique pour le ligand, et
   (c) si désiré, au moins un réactif choisi parmi des analogues du ligand et des partenaires de liaison spécifiques pour le ligand,
   l'un au moins de ces composants autres que l'échantillon étant marqué avec au moins un centre rédox choisi parmi le ferrocène et ses dérivés;
       lequel procédé comprend l'étape consistant à déterminer si, et, si cela est désiré, dans quelle mesure, une caractéristique électrochimique des composants dans une solution au repos est perturbée par au moins l'un des facteurs parmi (i) une formation de complexe et (ii) une influence externe contrôlée qui produit une perturbation de cette caractéristique électrochimique en fonction de cette formation de complexe.

2. Procédé suivant la revendication 1, dans lequel l'appareil électrochimique contient une électrode de travail solide.

3. Procédé suivant la revendication 1 ou la revendication 2, dans lequel l'influence externe contrôlée, lorsqu'elle est présente, comprend le déplacement du complexe formé par rapport à l'électrode de travail ou l'élimination du complexe de l'appareil.

4. Procédé suivant la revendication 2, dans lequel l'un des composants autres que l'échantillon est immobilisé sur l'électrode de travail.

5. Procédé suivant l'une quelconque des revendications 1, 2 et 4, dans lequel l'influence externe contrôlée, lorsqu'elle est présente, comprend le déplacement du composant modifié par un centre rédox non complexé en excès par rapport à l'électrode de travail de l'appareil électrochimique ou l'élimination du composant modifié par un centre rédox non complexé en excès de l'appareil.

6. Procédé suivant l'une quelconque des revendications précédentes, dans lequel le ou chaque composant modifié par un centre rédox contient au moins un centre rédox choisi parmi des dérivés fonctionnalisés du ferrocène.

7. Procédé suivant la revendication 6, dans lequel le dérivé fonctionnalisé du ferrocène contient une ou plusieurs chaînes latérales de formule -CHO, $-(CH_2)_nCOOH$ ou $-(CH_2)_mNH_2$ (où n et m sont chacun de 0 à 6).

16

8. Procédé suivant l'une quelconque des revendications précédentes, dans lequel la caractéristique électrochimique est une intensité de pic observée sous l'effet de l'application d'un potentiel présélectionné à travers les composants.

9. Procodé suivant l'une quelconque des revendications précédentes, dans lequel le ligand est un antigène ou un anticorps.

10. Kit pour réaliser un procédé d'essai suivant la revendication 1, qui comprend un réactif choisi parmi des partenaires de liaison spécifiques pour le ligand et des analogues du ligand marqués avec du ferrocène ou un dérivé de celui-ci, ce réactif étant immobilisé sur une électrode de travail solide.

11. Kit pour réaliser un procédé d'essai suivant la revendication 1, qui comprend un partenaire de liaison non marqué spécifique pour le ligand immobilisé sur un support solide et un autre réactif choisi parmi des analogues du ligand et des partenaires de liaison spécifiques pour le ligand marqués avec du ferrocène ou un dérivé de celui-ci.

**Patentansprüche**

1. Verfahren zur Untersuchung eines Liganden in einer Probe unter Verwendung einer elektrochemischen Apparatur, die Komponenten enthält, umfassend
   (a) die Probe,
   (b) einen spezifischen, sich an den Liganden bindenden Partner und
   (c) gewünschtenfalls zumindest ein Reagens, ausgewählt unter Ligandenanalogen und sich an den Liganden bindenden spezifischen Partnern,
   wobei zumindest eine dieser Komponenten, die von der Probe verschieden ist, mit zumindest einem Redox-Zentrum, ausgewählt unter Ferrocen und dessen Derivaten, markiert ist,

   welches Verfahren die Stufe der Bestimmung, ob und gewünschtenfalls in welchem Ausmaß ein elektrochemisches Merkmal der Komponenten in einer ruhenden Lösung durch zumindest eines von (i) Komplexbildung und (ii)einem kontrollierten äußeren Einfluß gestört wird, der eine Störung des elektrochemischen Merkmals als Funktion der Komplexbildung herbeiführt, umfaßt.

2. Verfahren gemäß Anspruch 1, bei dem die elektrochemische Apparatur eine feste Arbeitselektrode enthält.

3. Verfahren gemäß Anspruch 1 oder 2, bei dem der kontrollierte äußere Einfluß, wenn vorhanden, eine Verschiebung des gebildeten Komplexes in Bezug auf die Arbeitselektrode der elektrochemischen Apparatur oder die Entfernung des Komplexes aus der Apparatur umfaßt.

4. Verfahren gemäß Anspruch 2, bei dem eine der von der Probe verschiedenen Komponenten an der Arbeitselektrode immobilisiert wird.

5. Verfahren gemäß einem der Ansprüche 1, 2 und 4, bei dem der kontrollierte äußere Einfluß, wenn vorhanden, eine Verschiebung von überschüssiger, nicht-komplexierter, redoxmodifizierter Komponente in Bezug auf die Arbeitselektrode der elektrochemischen Apparatur oder die Entfernung von überschüssiger, nicht-komplexierter, redoxmodifizierter Komponente aus der Apparatur umfaßt.

6. Verfahren gemäß einem der vorhergehenden Ansprüche, bei dem die oder jede redoxmodifizierte Komponente zumindest ein Redox-Zentrum, ausgewählt unter funktionalisierten Derivaten des Ferrocens, umfaßt.

7. Verfahren gemäß Anspruch 6, bei dem das funktionalisierte Ferrocen-Derivat eine oder mehrere Seitenketten der Formeln -CHO, -(CH$_2$)$_n$COOH oder -(CH$_2$)$_m$NH$_2$ (worin n und m jeweils von 0 bis 6 betragen) enthält.

8. Verfahren gemäß einem der vorhergehenden Ansprüche, bei dem das elektrochemische Merkmal ein Spitzenstrom ist, der unter Anwendung eines vorgewählten Potentials durch die Komponenten hindurch beobachtet wird.

9. Verfahren gemäß einem der vorhergehenden Ansprüche, bei dem der Ligand ein Antigen oder ein Antikörper ist.

10. Ausrüstung zur Durchführung eines Untersuchungsverfahrens gemäß Anspruch 1, die ein Reagens umfaßt, ausgewählt unter spezifischen, sich an den Liganden und Ligandenanaloge, markiert mit Ferrocen oder einem Derivat hiervon, bindenden Partnern, wobei das Reagens an einer festen Arbeitselektrode immobilisiert ist.

11. Ausrüstung zur Durchführung eines Untersuchungsverfahrens gemäß Anspruch 1, das einen nicht-markierten, spezifischen, sich an den an einem festen Träger immobilisierten Liganden bindenden Partner und ein weiteres Reagens, ausgewählt unter Ligandenanalogen und spezifischen, sich an den mit Ferrocen oder einem Derivat hiervon markierten Liganden bindenden Partner, umfaßt.

# FIG. 1.

+310mV +410mV

0.1 μA

0       +255 mV        +750

Potential (mV vs SCE )

FIG.2a.

+320mV

0.1μA

0          +600

Potential (mV vs S.C.E.)

FIG.2b.

0.2μA

0                    +600

Potential (mV vs S.C.E.)

FIG. 3a.

FIG. 3b.

FIG.4.

FIG.5a.

0.1 μA    +350mV

+250mV

0          +600

Potential (mV vs S.C.E)

FIG. 5b.

0.1 μA

0          +600

Potential (mV vs S.C.E.)

# FIG. 6.